# EUROPEAN PATENT APPLICATION

(11) **EP 4 086 915 A1**
(43) Date of publication of application: **09.11.2022**
(21) Application number: 22171324.1
(22) Date of filing: 03.05.2022
(51) Int. Cl.: G16H 20/60, G16H 50/20

(54) **PERSONALIZED GLUCOSE RANGES FOR MAKING HEALTHY CHOICES**

(30) Priority: 05.05.2021 US 202163201583 P
(71) Applicant: ZOE Limited, London SE1 7RW (GB)
(72) Inventor: DAVIES, Richard James, London, SE1 7RW (GB)
(74) Representative: Patentgruppen A/S

(57) **Abstract**

Techniques are disclosed herein for generating personalized glucose ranges. In some examples, during a calibration period, a user may consume foods that they normally consume, as well as some standardized meals. Personalized upper limits and lower limits to the glucose range can be determined (e.g., using machine learning techniques) that account for the shape of glucose trace for the particular user, the food consumed during the calibration period, as well as other non-glucose factors such as questionnaires, the user's lipid profile, obesity or other health risks, and the like. In some cases, food recommendations may also be provided to the user (e.g., using machine learning techniques). The personalized glucose range and other information may be presented to the user on a display.

## Description

This application claims priority to U.S. Provisional Application No. 63/201,583, filed on May 5, 2021 and entitled "Personalized Glucose Ranges For Making Healthy Choices," the entire contents of which are incorporated herein by reference.

### Background

Determining healthy food choices for an individual can be challenging. Not only do individuals have a large variety of food choices, but food that is healthy for one individual may not be healthy for another individual. Age, sex, weight, the microbiome of an individual, as well as other factors affect what foods an individual should select to eat. For example, while low carbohydrate food or low-fat food may be beneficial for one individual, that same low carbohydrate or low-fat food choice may not be beneficial for another individual. In many cases, an individual may try many different types of foods and nutritional plans in an attempt to find the nutritional plan and foods that work best for them. Providing guidance about what to eat and how to change their diet can be very difficult and confusing for an individual given the plethora of food choices and the complex nature of the responses the individual has to different foods.

### Summary of the invention

The inventors have identified the above-mentioned problems and challenges related to individualized nutrition plans, choices and guidance, and subsequently made the below-described invention relating to generation of personalized glucose ranges, which may be used by an individual to help them understand how they can improve their diet, for example to readily determine how good/bad a particular food and/or meal is for them.

The invention relates to a method comprising accessing glucose data associated with a user, generating personalized glucose ranges, based at least in part on the glucose data, wherein the personalized glucose ranges are personalized for the user, and causing data associated with the personalized glucose ranges to be presented within a user interface to the user.

The glucose data may in an embodiment be received from a continuous glucose monitoring (CGM) device associated with the user. In an embodiment the glucose data is collected during a calibration period that lasts a number of days.

Nutritional data associated with the user may in an embodiment be received by the method, wherein the nutritional data indicates at least one food item that the user consumed during a calibration period. The calibration period may preferably be the same calibration period when the glucose data is collected.

Context data associated with the user may in an embodiment be received by the method, wherein the context data indicates at least one of a time of day of consumption, a previously eaten food item, an amount of exercise, and/or an amount of sleep associated with the user during a calibration period. In an embodiment the context data is received from the user via an application stored on a user device associated with the user.

A time of day in which a food item was eaten by the user, may in an embodiment be determined by the method, and the method may further be determining that a glucose level changes to at least one of above a first threshold value or below a second threshold value during the time of day, and associating the glucose level change with the food item in response to the glucose level changing when the food item was eaten.

At least one input identifying at least one food item may in an embodiment be received by the method, and the method may further be determining food data associated with the at least one food item, determining health data associated with the user, and generating a postprandial glucose response prediction associated with the at least one food item based at least in part on the food data and the health data. In an embodiment the method may be causing the postprandial glucose response prediction to be presented within a user interface to the user, for example in relation to their personalized glucose ranges. In an embodiment the method may be causing at least one biomarker score associated with the at least one food item based at least in part on the postprandial response prediction, to be presented within the user interface to the user, for example in relation to their personalized glucose ranges. In an embodiment the health data associated with the user are based at least in part on the personalized glucose ranges.

The food data may in an embodiment of the method include at least one of sugar content, carbohydrate content, glycemic index of the carbohydrate, fiber content, an amount of processing, or a food category. Health data may in an embodiment of the method include at least one of a blood glucose control score, a blood fat control score, or a gut health score.

The personalized glucose ranges may in an embodiment of the method further be based at least in part on accessing at least one of personalized health score data, single meal data, single food data, or combination of meal data.

In an embodiment the method is causing an indication of an upper limit and/or a lower limit of the glucose ranges to be presented via the user interface of the user. The data associated with the personalized glucose ranges may in an embodiment includes a line graph indicating a glucose level via a y-axis over a period of time via an x-axis. In an embodiment the indication to be presented via the user interface may be the mentioned line graph of glucose level over a period of time.

At least one weight may be applied to at least one of the health data in an embodiment of the method, wherein the at least one biomarker score is generated based at least in part on the at least one weight. In an embodiment of the method the at least one biomarker score is generated by a machine learning model.

The at least one biomarker score may in an embodiment of the method be generated based on accessing at least one of a family health history associated with the user, measures of blood chemistry taken in a fasting state associated with the user, or measures of blood chemistry that do not change postprandially associated with the user.

The invention relates to a system comprising one or more processors and a computer program comprising instructions that, when executed by the one or more processors, cause the one or more processors to perform the method according to any of the above. The invention relates to a computer program comprising instructions that, when executed by a computer, cause the computer to carry out the method according to any of the above.

The invention relates to a system comprising one or more processors, and one or more non-transitory computer-readable media comprising instructions that, when executed by the one or more processors, cause the one or more processors to perform operations comprising accessing glucose data associated with a user, generating personalized glucose ranges, based at least in part on the glucose data, wherein the personalized glucose ranges are personalized for the user, and causing data associated with the personalized glucose ranges to be presented within a user interface to the user.

The personalized glucose ranges are, in an embodiment of the system, further based at least in part on accessing at least one of personalized health score data, single meal data, single food data, or combination of meal data.

Nutritional data associated with the user may in an embodiment of the system configured with this feature, be received in the system, wherein the nutritional data indicates at least one food item that the user consumed during a calibration period. The system may in an embodiment further be configured to identify a glucose change associated with the user at a time in which the at least one food item was consumed. The system may further be configured to generate a biomarker score associated with the at least one food item based at least in part on the glucose change.

In an embodiment the system is configured to cause an indication of an upper limit and a lower limit of the glucose ranges to be presented via the user interface of the user. In an embodiment of the system, the data associated with the personalized glucose ranges includes a line graph indicating a glucose level via a y-axis over a period of time via an x-axis. This may in an embodiment be the indication of limits to be presented.

The invention relates to a method comprising accessing glucose data associated with a user, the glucose data being obtained at least partly from a continuous glucose monitor (CGM), generating personalized glucose ranges, based at least in part on the glucose data, wherein the personalized glucose ranges are personalized for the user; receiving at least one input identifying at least one food item, determining food data associated with the at least one food item, the food data including at least one of a sugar content, a carbohydrate content, a glycemic index of the carbohydrate, a fiber content, an amount of processing, or a food category, determining health data associated with the user based at least in part on the personalized glucose ranges, generating a postprandial response prediction associated with the at least one food item based at least in part on the food data and the health data, generating at least one biomarker score associated with the at least one food item based at least in part on the postprandial response prediction, and causing the at least one biomarker score to be presented within a user interface to the user. In an embodiment the method further comprises applying at least one weight to at least one of the health data, wherein the at least one biomarker score is generated based at least in part on the at least one weight. In an embodiment of the method, the at least one biomarker score is generated by a machine learning model. In an embodiment the method further comprises generating the at least one biomarker score based on accessing at least one of a family health history associated with the user, measures of blood chemistry taken in a fasting state associated with the user, or measures of blood chemistry that do not change postprandially associated with the user. In an embodiment the method further comprises receiving nutritional data associated with the user, wherein the nutritional data indicates at least one food item that the user consumed during a calibration period when the glucose data is collected.

### Brief description of the drawings

FIG 1. block diagram depicting an illustrative operating environment in which personalized glucose ranges are generated.
FIG. 2. is a block diagram depicting an illustrative operating environment in which personalized food guidance is generated using predicted food responses.
FIG. 3 is a block diagram depicting an illustrative operating environment in which a data ingestion service receives, and processes data associated with nutritional responses.
FIG. 4 illustrates a glucose trace from a continuous glucose monitoring (CGM) device.
FIG. 5 illustrates an example personalized glucose range adjustment from a fixed glucose range to a personalized glucose range on a healthy person.
FIG. 6 illustrates an example personalized glucose range adjustment from a fixed glucose range to a personalized glucose range on an un-healthy person.
FIG. 7 is a computer architecture diagram showing one illustrative computer hardware architecture for implementing a computing device that might be utilized to implement aspects of the various examples presented herein.
FIG. 8 illustrates a flowchart illustrating an example method and/or process for generating a personalized glucose range.
FIG. 9 illustrates a flowchart illustrating an example method and/or process for generating a biomarker score for a food item.

### Detailed description

The following detailed description is directed to technologies for generating personalized glucose ranges for making healthy choices. Using technologies described herein, instead of displaying glucose ranges that are not personalized for a user, personalized glucose ranges are generated based on various data, including but not limited to glucose traces recorded during a calibration period (e.g., a few days).

During the calibration period, the user may consume foods that they normally consume, as well as some standardized meals. Personalized upper limits and lower limits to the glucose range can be determined (e.g., using machine learning techniques) that account for the shape of glucose trace for the particular user, the food consumed during the calibration period, as well as other non-glucose factors such as questionnaires, the user's lipid profile, obesity or other health risks, and the like. The upper and lower limits may be updated over time. In some cases, food recommendations may also be provided to the user (e.g., using machine learning techniques). The personalized glucose range and other information may be presented to the user on a display.

In some examples, personalized food guidance may be provided that takes into account an individual's personalized responses to foods, such as the individual's glucose and fat responses, the individual's microbiome, the individual's overall health, potential health risks for the individual, and/or other data associated with the individual. Providing an individual with personalized food guidance can make choosing food easier and healthier and help them to understand how to improve their existing diet.

According to some examples, the personalized food guidance may include guidance data such as but not limited to personalized glucose ranges, personalized health scores (e.g., glucose, fat, cholesterol, etc.), guidance and/or recommendations on food that has not yet been eaten, and/or other guidance data (e.g., via a user interface (UI)) on food and/or meal(s) that have already been eaten). The guidance data may be used by an individual to help them understand how they can improve their diet. For instance, a user may receive guidance data by viewing a UI on a mobile device, or some other device, to readily determine how good and/or bad a particular food and/or meal is for them before they eat the food, how a particular food and/or meal was for them, how a particular food and/or meal is affected by food(s) already eaten by the user, and the like. The guidance data may also indicate a score, or some other indicator, that summarizes information about how the user has eaten over a particular time period (e.g., a day, a week, and the like).

To generate personalized food guidance, a nutritional service may produce personalized score(s) for one or more personalized health scores, and/or foods and/or meals that are based on personalized data about an individual and the individual's biological responses to the particular food and/or meals. The personalized data may include data that predicts how one or more of the individual's biomarkers will change postprandially, data that predicts an individual's potential risks such as diabetes or cardiovascular disease, as well other data associated with the individual.

The nutritional service may combine multiple data sources about the individual's response to each food in such a way that the nutritional service takes into account the individual's own personalized risks, so as to optimize the overall meal score for each individual based upon their own body's responses.

In some examples, the nutritional service may use one or more other services, to score the individual's responses to food on more than one dimension (e.g., the impact of the food on biomarkers such as glucose, triglycerides, insulin, energy and hunger and its impact on shifting the microbiome towards a specific goal). In some cases, these scores may depend on factors that change rapidly such as the time of day, what was eaten previously, the amount of exercise, and/or or the amount of sleep.

The nutritional service may also use other data when generating the scores for a particular food. For instance, the nutritional service may use properties of the food that can be measured independently of the user, such as grams of fiber, level of food processing, type of fiber, glycemic index, sugar content, carbohydrate content, glycemic index of the carbohydrate, salt content, alcohol content, whether the food is fermented, whether the food is a high polyphenol food such as dark chocolate or vegetables, whether the food is meat, the composition and source of fat in the food, the composition of the fiber in the food, the production method, preparation methods applied by the user (such as cooking method), the inclusion of particular ingredients such as (but not limited to) flavor enhancers or emulsifiers, the method by which the food was preserved, the location where the food was produced, the method by which animals contributing to the product were reared, caught or slaughtered, and the like. The nutritional service may weight these properties differently depending upon the individual (for example people with high lipid responses may be guided more strongly against alcohol and in favor of fiber).

Using techniques described herein, a personalized glucose range can be generated, based on each user's current state of health and eating habits, that gives a wide range of user's guidance to make healthy food choices.

In some examples, each user's personalized range can be determined from their glucose trace recorded during a calibration period (and/or other times), for example spanning a few days during which they adhere largely to their normal food habits perhaps including some standardized test meals. The user may be asked to record a timed food log during this calibration period, for analysis alongside their CGM trace, but other implementations may work based on CGM trace alone.

In some cases, the upper limit of the range can be set at a high percentile (e.g. 90th) of the recorded glucose levels from the calibration period, but it can also be determined using machine learning techniques that account for the shape of glucose trace and the actual meals logged by the user during the calibration period. Similarly, the lower limit can be set at a constant offset below the baseline glucose levels (for example 8.5% or 11% to detect glucose dips), but it can also be determined using machine learning techniques that account for other latent variables.

In some examples, both the lower and upper personalized limits may also be adjusted for non-glucose factors such as the user's lipid profile, obesity or other health risks. The personalized limits may also be controlled to stay within medical recommendations such as thresholds for diabetic hyperglycemia/hypoglycemia.

In some circumstances, multiple upper limits and lower limits may be shown to the user, giving an indication of how extreme an out of range response may be. For example a response might be above their 90th percentile but still below their 99th percentile. In some circumstances, only the upper or lower limit might be relevant for a user, so this can be presented as a single personalized glucose limit with an unbounded range at the other end, for example only showing a lower limit to highlight glucose dips.

In some examples, a personalized range may be tighter (e.g., smaller range) for a healthy user with a mostly healthy diet and may be wider (e.g., larger range) for an unhealthy user with a less healthy diet. This enables the personalized range to highlight a reasonable fraction of each of these user's meals, thereby enabling both of them to identify and avoid the unhealthiest foods in their current diet. In some cases, ranges may be selected so that a slightly higher fraction of meals are highlighted for the unhealthy user, but to a lesser extent than when using fixed glucose ranges. In addition to highlighting individual meals, summary metrics such as time in range or time out of range can be calculated for the user over a time period such as a day or week.

In some cases, a user may improve their diet to reduce glycemic load, peaks and dips whilst maintaining their current health, then it is expected that more of their meals will stay within their personalized glucose range - this effect can be seen immediately after the diet change, with fewer meals highlighted and more time in range. If a user's health improves while maintaining their current diet, then it is also expected that more of their meals will stay within their personalized glucose range - this effect may take longer since health improves over a longer time period. The number of individual meals highlighted and summary metrics such as time in range can therefore be used to track improvements in the user's diet and health over time.

In some examples, personalized glucose range can be recalibrated over time as a user's diet and health improve (or worsen). This can serve as a practical moving target that gets incrementally tighter as the user improves their diet and health, leading to continuous further improvement in their diet and health at a practical pace.

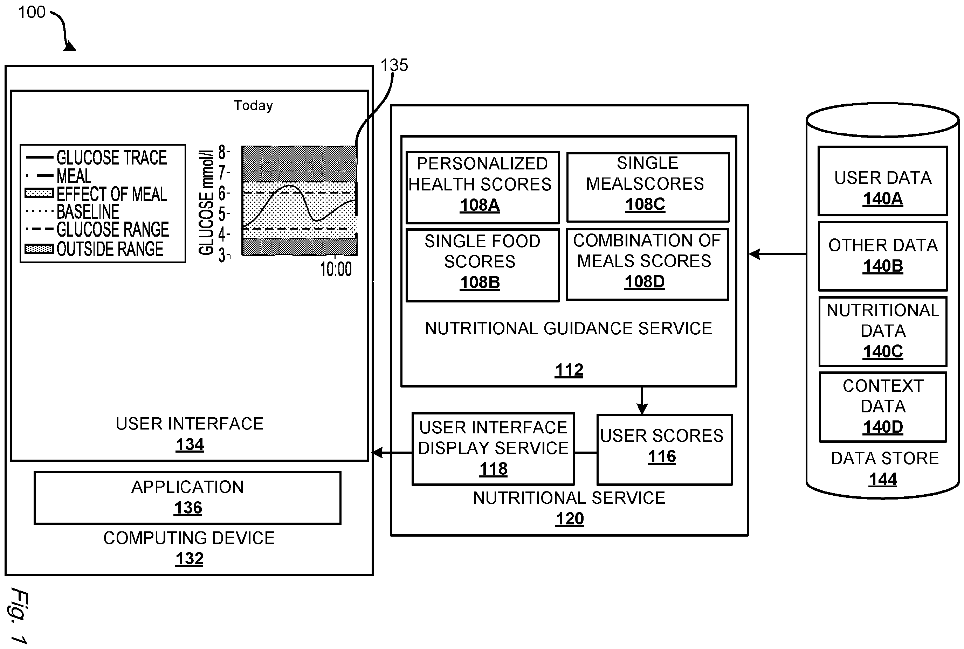

FIG. 1 is a block diagram depicting an illustrative operating environment 100 in which personalized glucose ranges are generated using predicted food responses. As illustrated, environment 100 includes a nutritional service 120, a nutritional guidance service 112, a UI display service 118, a data store 144, and a computing device 132.

As illustrated in FIG. 1, the operating environment 100 includes one or more computing devices 132 in communication with a nutritional service 120. In some examples, the environment 100 may be associated with and/or implemented by resources provided by a service provider network such as provided by a cloud computing company. The environment 100 may include a collection of computing resources (e.g., computing devices such as servers). The computing resources may include a number of computing, networking and storage devices in communication with one another. In some examples, the computing resources may correspond to physical computing devices and/or virtual computing devices implemented by one or more physical computing devices.

The data store 144 may include a variety of data 140, such as user data 140A, other data 140B, nutritional data 140C, and/or context data 140D. In some configurations, an individual (e.g., an individual associated with the computing device 132) may generate and provide user data 140A, such as health data 202 and test data 205) using a variety of at home biological collection devices, which collect a biological sample which requires a biological assay to be performed to generate the user data 140A. Some of the user data 140A may be biomarker data, such as blood glucose results collected by a continuous glucose monitor (CGM), as well as other types of data. In some cases, the other data 140B may include non-biomarker data such as photos, time stamps, as well as other data associated with a user or group of users.

Some of this data 140 may be nutritional data associated with individual foods, such as nutritional data 140C. In some configurations this may include data from nutritional databases and may include nutritional information such as fiber, amount of sugar, vitamin composition, vitamin amount, fat composition, fat amount, and the like. Some of this data may come from individuals who have recorded eating such food and shared their own biological responses to this food such as blood glucose responses or reported hunger. By aggregating this data and using other information about these individuals, in some configurations some of the nutritional data for this food such as its impact on blood sugar can be calculated.

Some of this data 140 may be context data associated with a context in which food was consumed, such as context data 140D. For example, the context data 140D may include information received from the individual consuming the food items (and/or another individual on behalf of the individual consuming the food items) and may include a time of day of consumption, a previously eaten food item, an amount of exercise and/or an amount of sleep associated with the individual during a calibration period.

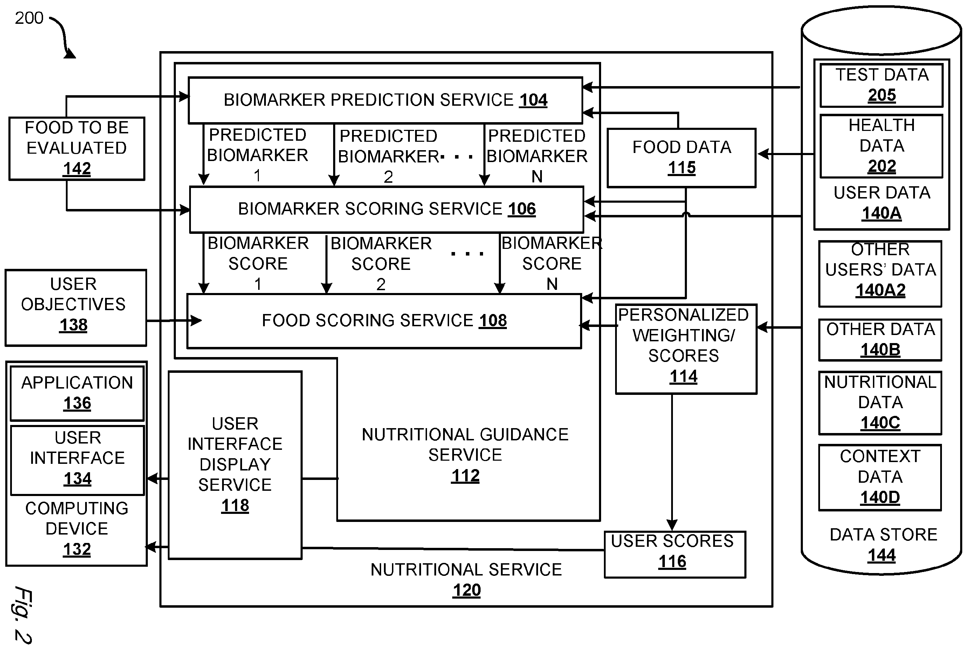

An individual may also provide data 140 using computing device 132. In some configurations an individual can input data 140 into one or more software applications 136. For example, an individual may enter the food they consumed, a value indicated by a measurement device, their waist measurement, and the like. Alternatively, and/or in addition to the above, data 140 generated by other measurements can be used to assist in determining when a food was eaten, and/or a test was performed. For example, in some cases a CGM can monitor glucose levels and be used to confirm the start point of a meal. In this example, data recorded by an individual about when they started to eat can be verified by confirming that there is a rise in glucose detected by the CGM, provided there was sufficient carbohydrate in the meal. More details on the type of data that may be included in data store 144 is presented with the description of FIG. 2.

In some cases, the nutritional service 120 can access the data 140 when generating the personalized nutritional guidance and/or scores for a particular user. In some examples, the nutritional service 120 utilizes both data associated with the user providing the data and data from other users performing similar tests. In other examples, the data utilized is associated only with the user. According to some examples, the data 140 can include data obtained from a clinical setting, which may be more accurate than at home measurements.

In some examples, the nutritional service 120 may utilize one or more machine learning mechanisms. For example, the nutritional service 120 can use a classifier to classify the data within a classification category. In other examples, the nutritional service 120 may generate one or more scores using one or more machine learning mechanisms.

As briefly discussed above, the nutritional service 120 generates personalized food guidance using predicted food responses. Instead of providing a user, such as a user of computing device 132 with food guidance that is generalized for a group of individuals, the nutritional service 120 generates personalized food guidance that takes into account the user's responses to foods, such as the individual's glucose and fat responses, the individual's microbiome, the individual's overall health, potential health risks for the individual, and/or other user data 140B that is associated with the individual.

According to some configurations, the nutritional service 120 uses a nutritional guidance service 112 to generate a variety of personalized food guidance data that may be provided to a user via UI 134, or through some other mechanism. The personalized food guidance data may include data such as but not limited to personalized health scores (e.g., glucose, fat, cholesterol, etc.) 108A, food item scores 108B (e.g., guidance / recommendations / scores on single food items that have already been eaten or not yet eaten), single meal scores 108C (guidance / recommendations / scores on single meals that have already been eaten or not yet eaten), combination of meal scores 108D (guidance/recommendations/scores on combinations of meals that have already been eaten or not yet eaten), and/or other guidance data (not shown).

In some examples, the nutritional guidance service 112 generates user scores 116 (e.g., guidance data) and provides the user scores 116 to a user interface display service 118 to provide to the user via UI 134. The user scores 116 displayed in UI 134 may be used by an individual to help them understand how they can improve their diet. For instance, a user may receive the user scores 116 by viewing UI 134 on a mobile device, or some other device, to readily determine how good/bad a particular food and/or meal is for them. In the current example illustrated in FIG. 1, instead of just showing the user a glucose trace that has non-personalized ranges, the nutritional guidance service 112 generates personalized glucose ranges for a user. As illustrated, the UI 134 shows a portion of a glucose trace for a user along with personalized glucose ranges.

In some examples, the user scores 116 may include the user's personalized range determined from their glucose trace recorded during a calibration period (and/or other times), for example spanning a few days during which they adhere largely to their normal food habits perhaps including some standardized test meals. The user may be asked to record a timed food log during this calibration period, for analysis alongside their CGM trace, but other implementations may work based on CGM trace alone.

In some cases, the user scores 116 may include a graph 135 illustrating an upper limit and a lower limit of the glucose range. In some examples, the upper limit of the range can be set at a high percentile (e.g. 90th) of the recorded glucose levels from the calibration period, but it can also be determined using machine learning techniques that account for the shape of glucose trace and the actual meals logged by the user during the calibration period. Similarly, the lower limit can be set at a constant offset below the baseline glucose levels (for example 8.5% or 11% to detect glucose dips), but it can also be determined using machine learning techniques that account for other latent variables.

In some examples, both the lower and upper personalized limits may also be adjusted for non-glucose factors such as the user's lipid profile, obesity or other health risks. The personalized limits may also be controlled to stay within medical recommendations such as thresholds for diabetic hyperglycemia/hypoglycemia.

In some circumstances, multiple upper limits and lower limits may be shown to the user, giving an indication of how extreme an out of range response may be. For example a response might be above their 90th percentile but still below their 99th percentile. In some circumstances, only the upper or lower limit might be relevant for a user, so this can be presented as a single personalized glucose limit with an unbounded range at the other end, for example only showing a lower limit to highlight glucose dips.

In some examples, a personalized range may be tighter (e.g., smaller range) for a healthy user with a mostly healthy diet and may be wider (e.g., larger range) for an unhealthy user with a less healthy diet. This enables the personalized range to highlight a reasonable fraction of each of these user's meals, thereby enabling both of them to identify and avoid the unhealthiest foods in their current diet. In some cases, ranges may be selected so that a slightly higher fraction of meals are highlighted for the unhealthy user, but to a lesser extent than when using fixed glucose ranges. In addition to highlighting individual meals, summary metrics such as time in range or time out of range can be calculated for the user over a time period such as a day or week.

In some examples, personalized glucose range can be recalibrated over time as a user's diet and health improve (or worsen). This can serve as a practical moving target that gets incrementally tighter as the user improves their diet and health, leading to continuous further improvement in their diet and health at a practical pace.

FIG. 2 is a block diagram depicting an illustrative operating environment 200 in which personalized food guidance is generated using predicted food responses. As illustrated, environment 200 includes more details as compared to environment 100. For example, environment 200 includes biomarker prediction service 104, biomarker scoring service 106, food scoring service 108 as part of the nutritional guidance service 112. In other configurations, the services may be located in different environments and/or locations.

In some examples, the nutritional guidance service 112 may receive one or more inputs (e.g., the glucose rise linked to foods). In this example, the nutritional guidance service 112 generates a prediction of the biomarker of interest using biomarker prediction service 104 and a score for that biomarker using the biomarker scoring service 106. However, in other examples where more than one aspect of the food is calculated, nutritional guidance service 112 predicts many different biomarkers (e.g., biomarkers 1-N) of the assessed aspects (glucose response, blood fat response, and the like), scores each biomarker change individually using the biomarker scoring service 106 and weights the scores so that the scores are comparable and then combined into a single score using food scoring service 108.

In some examples, in addition to utilizing biomarker inputs, the nutritional guidance service 112 also utilizes non-biomarker inputs such as but not limited to food data 115 such as sugar content, carbohydrate content, glycemic index of the carbohydrate, fiber content, amount of processing on the food (e.g., high, medium, low), food category and the like. In some examples the nutritional guidance service uses information about the individual to determine personalizing weighting 114 for combining these scores, which determine how to combine the different biomarker scores of the biomarker scoring service 106 and food data, 115 into a single score.

The biomarker prediction service 104 generates a prediction of the postprandial response of the user's biomarker to the food 142 being evaluated. The biomarker scoring service 106 generates scores that are personalized for a particular user. The scores generated by the biomarker scoring service 106 are related to the predicted health impact of these biomarker changes on the particular user. For example, a small rise in blood fat after a meal might have no negative impact on health and so the score for blood fat might be at the best level for that meal but a large rise in blood fat 6 hours after a meal might be detrimental to health and lead to a bad score for blood fat for that meal for that person. The nutritional service 120 may then use these biomarker scores to generate food scores for one or more foods/combination of foods and/or meals to make individual food recommendations by generating personalized meal scores for any meal using food scoring service 108. In some examples, the food scoring service 108 uses personalized weighting/scores 114 to determine how to weight the different biomarker scores when combining them to generate a single meal score. For example, the food scoring service might need to combine a blood glucose score, a blood fat score and a gut health score, and might do so by weighting each of these scores and then combining them.

In some examples, the nutritional service 120 may use one or more other services, to score the individual's responses to food on more than one dimension, such as its impact on shifting the microbiome towards a specific goal, based on user objectives 138. For example, the food scoring service 108 may account for one or more user objectives 138 received by the user, and/or on behalf of the user (e.g., by a health professional), indicating one or more user objectives, such as but not limited to, weight loss, cholesterol decrease, glucose level normalization, blood pressure normalization, etc.

In some examples, if the personalized impact of a food (e.g., food 142) is to be calculated by using predictions of the glucose and triglycerides levels after eating that food combined with the food's impact on the microbiome for that individual, generating a meal score by the food scoring service 108 may consist of using the personalized weighting/scores 114 to combine all three of these scores. The nutritional service 120 is designed to do this such that it recommends the best possible food for the individual based upon the inputs provided. In other examples the nutritional service 120 will use information beyond biomarker predictions as inputs into its biomarker scoring, for example it may take into account family health history, measures of blood chemistry taken in a fasting state such as baseline glucose or triglycerides, or measures of blood chemistry that do not change postprandially such as cholesterol or HbA1c. The nutritional service 120 can also utilize one or more data analysts, machine learning, and/or some other mechanism to generate a quality score for the data.

In some examples, the personalized impact of the food 142 being evaluated and any associated recommendation may be in the form of the user scores 116 and provided, via the UI display service 118, to the UI 134 and/or the application 136 of the computing device 132 for presentation to the user.

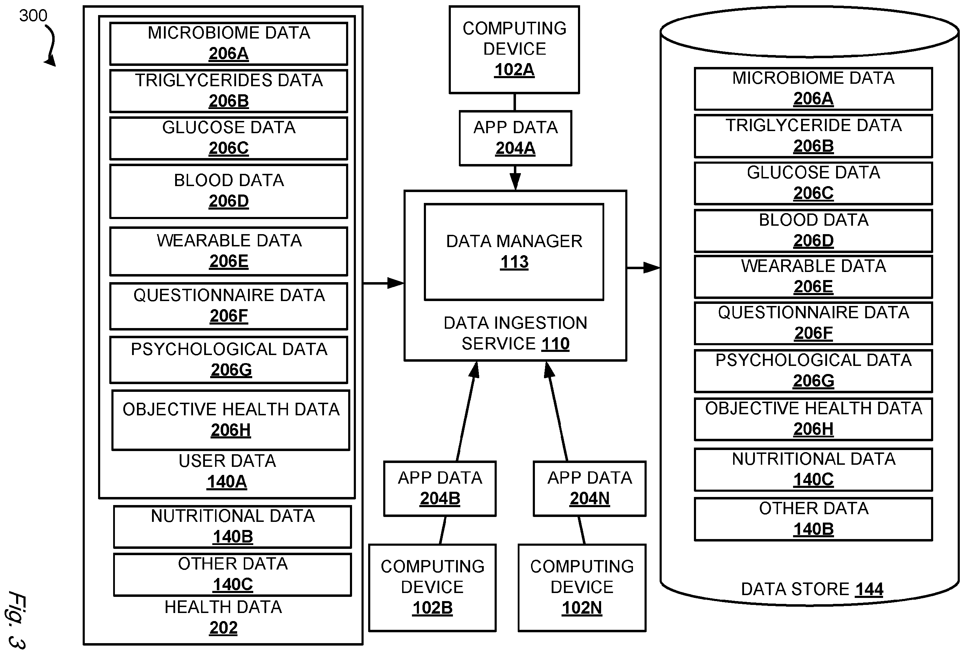

FIG. 3 is a block diagram depicting an illustrative operating environment 300 in which a data ingestion service 110 and data manager 113 receives and processes data associated with nutritional responses. The data ingestion service 110 and/or the data manager 113 may be a component and/or a subcomponent of the nutritional service 120. In other examples, the data ingestion service 110 and/or the data manager 113 may be remote component operating within the environment 100 and/or the environment 200.

In some configurations, the data manager 113 is configured to receive data such as, health data 202 that can include, but is not limited to microbiome data 206A, triglycerides data 206B, glucose data 206C, blood data 206D, wearable data 206E (e.g., circadian data, time awake data, etc.), questionnaire data 206F, psychological data 206G (e.g., hunger, sleep quality, mood, etc.), objective health data 206H (e.g., height, weight, medical history, etc.), nutritional data 140C, and other data 140C.

According to some examples, the microbiome data 206A includes data about the gut microbiome of an individual. The gut microbiome can host a large number of microbial species (e.g., > 1000) that together have millions of genes.

The triglycerides data 206B may include data about triglycerides for an individual. In some examples, the triglycerides data 206B can be determined from an At Home Blood Test which in some cases is a finger prick on to a dried blood spot card. The glucose data 206C includes data about blood glucose. The glucose data 206C may be determined from various testing mechanisms, including at home measurements, such as a continuous glucose meter.

The blood data 206D may include blood tests relating to a variety of different biomarkers. As discussed above, at least some blood tests can be performed at home. In some configurations, the blood data 206D is associated with measuring blood sugar, insulin, c-peptides, triglycerides, cholesterol, IL-6 inflammation, ketone bodies, nutrient levels, allergy sensitivities, iron levels, blood count levels, HbA1c, and the like.

The wearable data 206E can include any data received from a computing device associated with an individual. For instance, an individual may wear an electronic data collection device, such as an activity-monitoring device, that monitors motion, heart rate, heart rate variability, determines how much an individual has slept that may include the types of sleep, the times an individual is awake, the number of calories burned, activities performed, blood pressure, body temperature, and the like. The individual may also wear a continuous glucose meter that monitors blood glucose levels.

The questionnaire data 206F can include data received from one or more questionnaires, and/or surveys received from one or more individuals. The psychological data 206G, that may be subjectively obtained, may include data received from the individual and/or a computing device that generates data or input based on a subjective determination (e.g., the individual states that they are still hungry after a meal, or a device estimates sleep quality based on the movement of the user at night perhaps combined with heart rate data). The objective health data 206H includes data that can be objectively measured, such as but not limited to height, weight, medical history, and the like.

The nutritional data 140C can include data about food, which is referred to herein as "food data". For example, the nutritional data can include nutritional information about different food(s) such as their macronutrients and micronutrients or the bioavailability of its nutrients under different conditions (raw vs cooked, or whole vs ground up).

The other data 140B can include other data associated with the individual. For example, the other data 140B can include data that can be received directly from a computer application that logs information for an individual (e.g., food eaten, sleep, ..) and/or from the user via a user interface.

In some examples, different computing devices 102 associated with different users provide application data 204 to the data manager 113 for ingestion by the data ingestion service 110. As illustrated, computing device 102A provides app data 204A to the data manager 113, computing device 102B provides app data 204B to the data manager 113, and computing device 102N provides app data 204N to the data manager 113. There may be any number of computing devices utilized.

As discussed briefly above, the data manager 113 receives data from different data sources, processes the data when needed (e.g., cleans up the data for storage in a uniform manner), and stores the data within one or more data stores, such as the data store 144.

The data manager 113 can be configured to perform processing on the data before storing the data in the data store 144. For example, the data manager 113 may receive data for ketone bodies and then use that data to generate ketone body ratios. Similarly, the data manager 113 may process food eaten and generate meal calories, number of carbohydrates, fat to carbohydrate ratios, how much fiber consumed during a time period, and the like. The data stored in the data store 144, or some other location, can be utilized by the nutritional service 120 to determine an accuracy of at home measurements of nutritional responses performed by users. The data outputted by the data manager 113 to the nutritional service 120 may therefore contain different values than are stored in the data store 144, for example if a food quantity is adjusted.

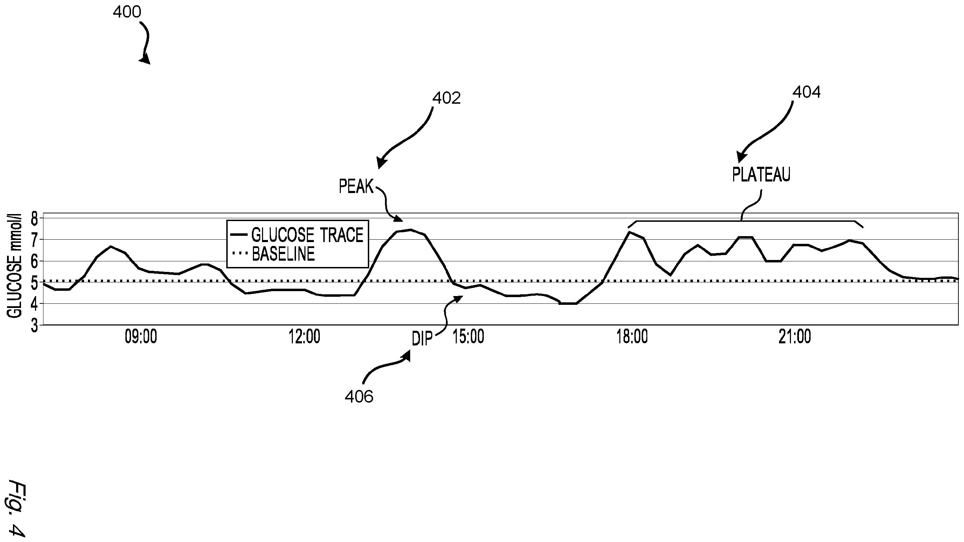

FIG. 4 illustrates an example glucose trace 400 from a CGM that does not include an upper or lower limit value. The features of the glucose trace 400 include a peak 402 (e.g. sudden increase in glucose levels after a meal immediately followed by a sharp decrease), a plateau 404 (e.g., period of sustained high levels of glucose after a meal), and a dip 406 (e.g., a period of low levels of glucose below the baseline (average glucose level at rest before meals), typically in the 2 to 3 hours after a meal.

In some cases, peak/plateaus may cause diabetic hyperglycemia but also longer-term health impacts including oxidative stress, dietary inflammation and cumulative stress on insulin generating beta cells in the pancreas. In some cases, dips may cause diabetic hypoglycemia but also longer term health impacts including low perceived energy, increased perceived hunger and increased subsequent calorie consumption.

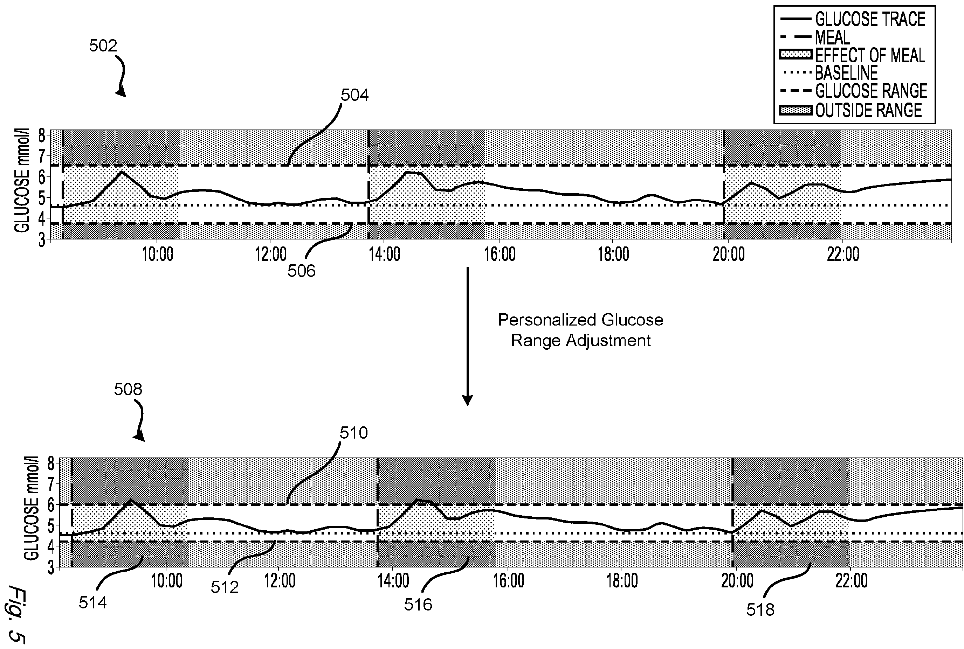

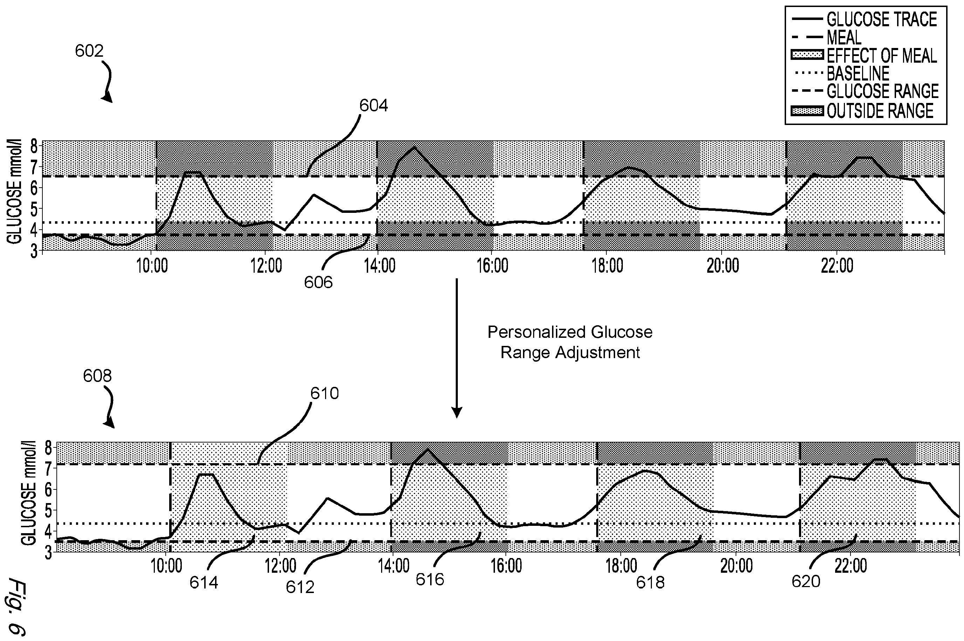

FIGS. 5 and 6 illustrate example glucose trace 502 for a healthy user including a fixed upper limit 504 and fixed lower limit 506 as well as glucose trace 602 for an unhealthy user including a fixed upper limit 604 and fixed lower limit 606, typically determined either from diabetic hyperglycemia/hypoglycemia, generic medical advice such as ADA recommendations, or from the average glucose range of healthy individuals in the population. These fixed ranges are not personalized to an individual or their diet. Such fixed ranges tend to be wide owing to the high degree of interpersonal variations observed in healthy individuals to different meals. So, it is likely that an average healthy user (e.g., healthy user that has good glycemic control) might rarely stray out of this non-personalized glucose range even while consuming unhealthy meals (e.g., unhealthy meals may include high glycemic load), as illustrated in the glucose trace 502 and glucose trace 602.

In some examples, a personalized glucose range adjustment, generated by the techniques discussed herein, may generate a personalized glucose range illustrated by the glucose trace 508 having a personalized upper limit 510 and a personalized lower limit 512 as well as the glucose trace 608 having a personalized upper limit 610 and a personalized lower limit 612. For example, each user's personalized range can be determined from their glucose trace recorded during a calibration period (and/or other times), for example spanning a few days during which they adhere largely to their normal food habits perhaps including some standardized test meals. The user may be asked to record a timed food log during this calibration period, for analysis alongside their CGM trace, but other implementations may work based on CGM trace alone.

The upper limit of the range can be set at a high percentile (e.g. 90th) of the recorded glucose levels from the calibration period, but it can also be determined using machine learning techniques that account for the shape of glucose trace and the actual meals logged by the user during the calibration period.

Similarly, the lower limit can be set at a constant offset below the baseline glucose levels (for example 8.5% or 11% to detect glucose dips), but it can also be determined using machine learning techniques that account for other latent variables.

Both the lower and upper personalized limits may also be adjusted for non-glucose factors such as the user's lipid profile, obesity or other health risks. The personalized limits may also be controlled to stay within medical recommendations such as thresholds for diabetic hyperglycemia/hypoglycemia.

In some circumstances, multiple upper limits and lower limits may be shown to the user, giving an indication of how extreme an out of range response is - for example a response might be above their 90th percentile but still below their 99th percentile. In some circumstances, only the upper or lower limit might be relevant for a user, so this can be presented as a single personalized glucose limit with an unbounded range at the other end, for example only showing a lower limit to highlight glucose dips.

In some examples, a personalized range will be tighter for a healthy user with a mostly healthy diet, while in some cases, it will be wider for an unhealthy user with a less healthy diet. This enables the personalized range to highlight a reasonable fraction of each of these user's meals, thereby enabling both of them to identify and avoid the unhealthiest foods in their current diet. Ranges might be selected so that a slightly higher fraction of meals are highlighted for the unhealthy user, but to a lesser extent than when using fixed glucose ranges. In addition to highlighting individual meals (e.g., highlighted portions 514, 516, 518, 614, 616, 618, and 620), summary metrics such as time in range or time out of range can be calculated for the user over a time period such as a day or week. As illustrated in FIGS. 4-6, the data associated with the personalized glucose ranges may include a line graph indicating a glucose level via a y-axis over a period of time via an x-axis.

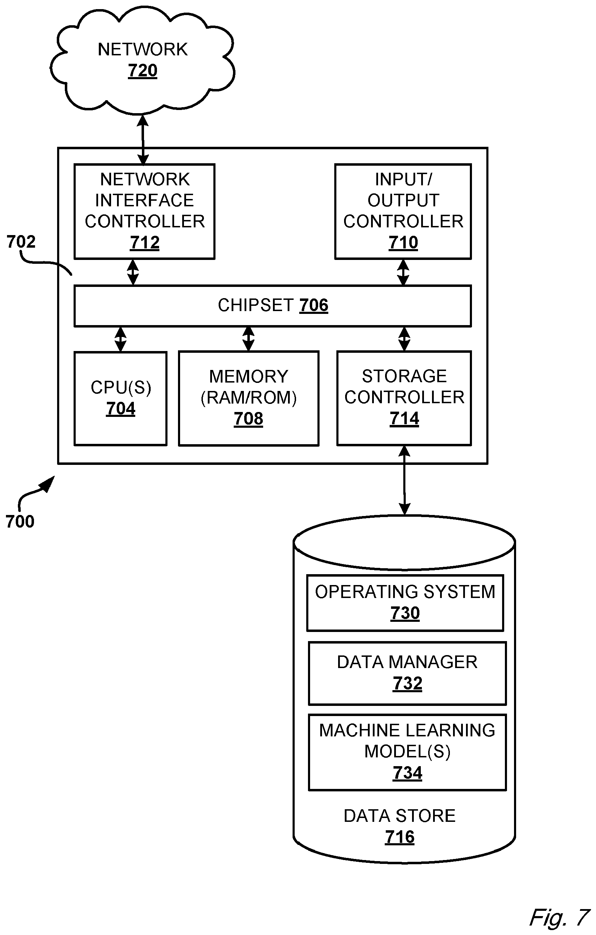

FIG. 7 shows an example computer architecture for a computer 700. The computer architecture shown in FIG. 7 illustrates a conventional server computer, workstation, desktop computer, laptop, mobile device, tablet, network appliance, digital cellular phone, smart watch, or other computing device, and may be utilized to execute any of the software components presented herein. For example, the computer architecture shown in FIG. 7 may be utilized to execute software components for performing operations as described above with regard to the environments 100, 200, and/or 300. The computer architecture shown in FIG. 7 might also be utilized to implement a computing device 132, or any other of the computing systems described herein.

The computer 700 includes a baseboard 702, or "motherboard," to which a multitude of components or devices may be connected by way of a system bus or other electrical communication paths. In one illustrative example, one or more central processing units ("CPUs") 704 operate in conjunction with a chipset 706. The CPUs 704 may be standard programmable processors that perform arithmetic and logical operations necessary for the operation of the computer 700.

The chipset 706 provides an interface between the CPUs 704 and the remainder of the components and devices on the baseboard 702. The chipset 706 may provide an interface to memory 708, such as RAM and/or read-only memory ("ROM"). The memory 708 may store software components utilized for the operation of the computer 700 in accordance with the examples described herein.

The computer 700 may operate in a networked environment using logical connections to remote computing devices and computer systems through a network, such as the network 720. The chipset 706 may include functionality for providing network connectivity through a network interface controller, such as a cellular network adapter, WiFi network adapter, Ethernet adapter, and the like. The NIC 712 is capable of connecting the computer 700 to other computing devices over the network 720.

The computer 700 may be connected to a mass storage device 716 that provides storage for the computer. The mass storage device 716 may store system programs, application programs, other program modules and data, which have been described in greater detail herein. The mass storage device 716 may be connected to the computer 700 through a storage controller 714 connected to the chipset 706. The mass storage device 716 may consist of one or more physical storage units. The storage controller 714 may interface with the physical storage units through various type of interfaces.

The mass storage device 716 described above, the computer 700 may have access to other computer-readable storage media to store and retrieve information, such as program modules, data structures, or other data. By way of example, and not limitation, computer-readable storage media may include volatile and non-volatile, removable and non-removable media implemented in any method or technology.

The mass storage device 716 may store an operating system 730 utilized to control the operation of the computer 700. According to some configurations, the operating system may include, but is not limited to the UNIX operating system, the LINUX operating system, the WINDOWS^{®} operating system from MICROSOFT Corporation, the iOS operating system from APPLE Corporation, the ANDROID operating system from GOOGLE, and the like. Other operating systems may also be utilized. The mass storage device 716 may store other system or application programs and data utilized by the computer 700, such as components that include the data manager 732 (which may be the same or similar to the data manager 113), and/or any of the other software components and data described above. The mass storage device 716 might also store other programs and data not specifically identified herein.

In one example, the mass storage device 716 or other computer-readable storage media is encoded with computer-executable instructions that, when loaded into the computer 700, implement the examples described herein. The computer 700 has access to computer-readable storage media storing computer-executable instructions which, when executed by the computer 700, may be configured to perform the various routines described above. The computer 700 might also include computer-readable storage media for performing any of the other computer-implemented operations described herein.

The mass storage device 716 may store one or more machine learning models 734 utilized to determine one or more values described herein. For example, depending on the value to be determined, the machine learning model 734 may be generated that is configured to determine the value at issue. For example, the machine learning model 734 may be configured to intake, as input, data the machine learning model 734 is configured to utilize and to perform one or more operations to determine the value. Additionally, a training dataset may be generated and utilized to train the machine learning model 734 such that a trained machine learning model 734 is generated. The trained machine learning model 734 may be utilized to determine the values described herein. Just by way of example, training of the machine learning models 734 may result in determining the one or more weighting values (e.g., associated with the health data, food data, biomarker scores, and/or user scores, etc.) health data, food data, biomarker scores, user scores (e.g., upper limit values, lower limit values, food recommendations, etc.), user data, nutritional data, other data, etc..

Predictive analytic techniques may include, for example, predictive modelling, machine learning, and/or data mining. Generally, predictive modelling may utilize statistics to predict outcomes. Machine learning, while also utilizing statistical techniques, may provide the ability to improve outcome prediction performance without being explicitly programmed to do so. A number of machine learning techniques may be employed to generate and/or modify the layers and/or models describes herein. Those techniques may include, for example, decision tree learning, association rule learning, artificial neural networks (including, in examples, deep learning), inductive logic programming, support vector machines, clustering, Bayesian networks, reinforcement learning, representation learning, similarity and metric learning, sparse dictionary learning, and/or rules-based machine learning.

Information from stored and/or accessible data may be extracted from one or more databases, and may be utilized to predict trends and behavior patterns. The predictive analytic techniques may be utilized to determine associations and/or relationships between explanatory variables and predicted variables from past occurrences and utilizing these variables to predict the unknown outcome. The predictive analytic techniques may include defining the outcome and data sets used to predict the outcome.

Data analysis may include using one or more models, including for example one or more algorithms, to inspect the data with the goal of identifying useful information and arriving at one or more determinations that assist in predicting the outcome of interest. One or more validation operations may be performed, such as using statistical analysis techniques, to validate accuracy of the models. Thereafter predictive modelling may be performed to generate accurate predictive models.

The computer 700 may also include one or more input/output controllers 710 for receiving and processing input from a number of input devices, such as an electronic data collection device, a keyboard, a mouse, a touchpad, a touch screen, an electronic stylus, or other type of input device. Similarly, the input/output controller 710 may provide output to one or more types of output devices (e.g., a display, a projector, a printer, ...). The computer 700 may not include all of the components shown in FIG. 7, may include other components that are not explicitly shown in FIG. 7, or may utilize an architecture completely different than that shown in FIG. 7.

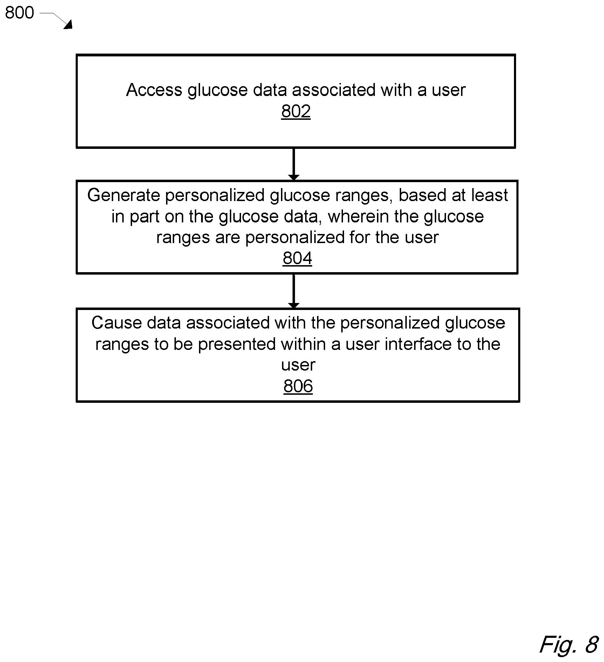

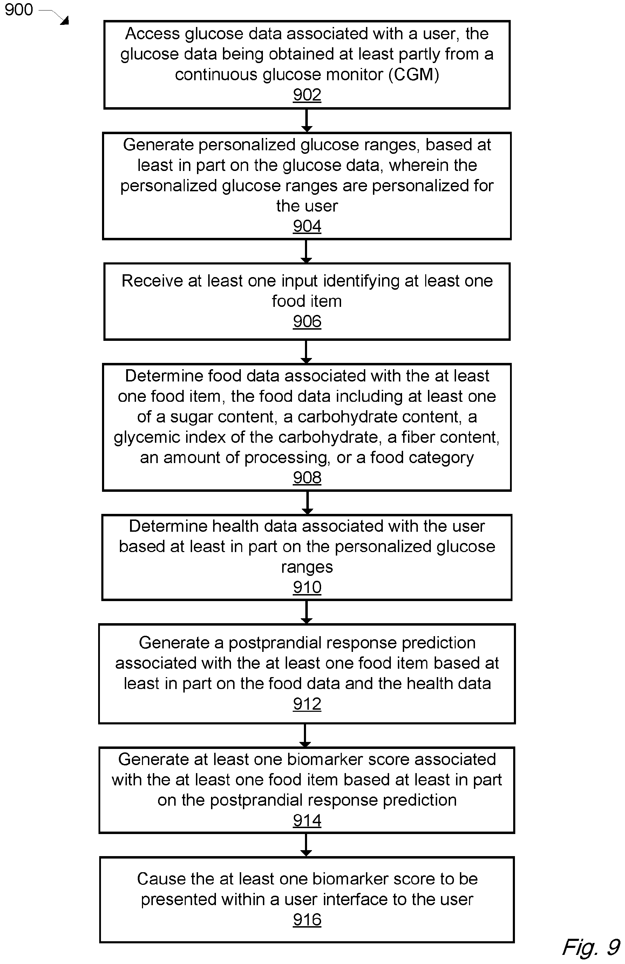

FIGS. 8 and 9 illustrate processes associated with generating personalized glucose ranges and food recommendations. The processes described herein are illustrated as collections of blocks in logical flow diagrams, which represent a sequence of operations, some or all of which may be implemented in hardware, software or a combination thereof. In the context of software, the blocks may represent computer-executable instructions stored on one or more computer-readable media that, when executed by one or more processors, program the processors to perform the recited operations. Generally, computer-executable instructions include routines, programs, objects, components, data structures and the like that perform particular functions or implement particular data types. The order in which the blocks are described should not be construed as a limitation, unless specifically noted. Any number of the described blocks may be combined in any order and/or in parallel to implement the process, or alternative processes, and not all of the blocks need be executed. For discussion purposes, the processes are described with reference to the environments, architectures and systems described in the examples herein, such as, for example those described with respect to FIGS. 1-7, although the processes may be implemented in a wide variety of other environments, architectures and systems.

FIG. 8 illustrates a flow diagram of an example process 800 for generating personalized glucose ranges. The order in which the operations or steps are described is not intended to be construed as a limitation, and any number of the described operations may be combined in any order and/or in parallel to implement process 800. The operations described with respect to the process 800 are described as being performed by a nutritional service. However, it should be understood that some or all of these operations may be performed by some or all of components, devices, and/or systems described herein.

At block 802, the process 800 may include accessing glucose data associated with a user. For example, the data store 144 may include a variety of data 140, such as user data 140A, other data 140B, nutritional data 140C, and/or context data 140D. In some configurations, an individual (e.g., an individual associated with the computing device 132) may generate and provide user data 140A, such as health data 202 and test data 205) using a variety of at home biological collection devices, which collect a biological sample which requires a biological assay to be performed to generate the user data 140A. Some of the user data 140A may be biomarker data, such as blood glucose results collected by a continuous glucose monitor (CGM), as well as other types of data. In some cases, the other data 140B may include non-biomarker data such as photos, time stamps, as well as other data associated with a user or group of users.

At block 804, the process 800 may include generating personalized glucose ranges, based at least in part on the glucose data, wherein the glucose ranges are personalized for the user. For example, the nutritional guidance service 112 generates user scores 116 (e.g., guidance data) and provides the user scores 116 to a user interface display service 118 to provide to the user via UI 134. The user scores 116 displayed in UI 134 may be used by an individual to help them understand how they can improve their diet. For instance, a user may receive the user scores 116 by viewing UI 134 on a mobile device, or some other device, to readily determine how good/bad a particular food and/or meal is for them. In the current example illustrated in FIG. 1, instead of just showing the user a glucose trace that has non-personalized ranges, the nutritional guidance service 112 generates personalized glucose ranges for a user. As illustrated, the UI 134 shows a portion of a glucose trace for a user along with personalized glucose ranges.

In some examples, the user scores 116 may include the user's personalized range determined from their glucose trace recorded during a calibration period (and/or other times), for example spanning a few days during which they adhere largely to their normal food habits perhaps including some standardized test meals. The user may be asked to record a timed food log during this calibration period, for analysis alongside their CGM trace, but other implementations may work based on CGM trace alone.

In some cases, the user scores 116 may include a graph 135 illustrating an upper limit and a lower limit of the glucose range. In some examples, the upper limit of the range can be set at a high percentile (e.g. 90th) of the recorded glucose levels from the calibration period, but it can also be determined using machine learning techniques that account for the shape of glucose trace and the actual meals logged by the user during the calibration period. Similarly, the lower limit can be set at a constant offset below the baseline glucose levels (for example 8.5% or 11% to detect glucose dips), but it can also be determined using machine learning techniques that account for other latent variables.

In some examples, both the lower and upper personalized limits may also be adjusted for non-glucose factors such as the user's lipid profile, obesity or other health risks. The personalized limits may also be controlled to stay within medical recommendations such as thresholds for diabetic hyperglycemia/hypoglycemia.

In some circumstances, multiple upper limits and lower limits may be shown to the user, giving an indication of how extreme an out of range response may be. For example a response might be above their 90th percentile but still below their 99th percentile. In some circumstances, only the upper or lower limit might be relevant for a user, so this can be presented as a single personalized glucose limit with an unbounded range at the other end, for example only showing a lower limit to highlight glucose dips.

In some examples, a personalized range may be tighter (e.g., smaller range) for a healthy user with a mostly healthy diet and may be wider (e.g., larger range) for an unhealthy user with a less healthy diet. This enables the personalized range to highlight a reasonable fraction of each of these user's meals, thereby enabling both of them to identify and avoid the unhealthiest foods in their current diet. In some cases, ranges may be selected so that a slightly higher fraction of meals are highlighted for the unhealthy user, but to a lesser extent than when using fixed glucose ranges. In addition to highlighting individual meals, summary metrics such as time in range or time out of range can be calculated for the user over a time period such as a day or week.

In some examples, personalized glucose range can be recalibrated over time as a user's diet and health improve (or worsen). This can serve as a practical moving target that gets incrementally tighter as the user improves their diet and health, leading to continuous further improvement in their diet and health at a practical pace.

At block 806, the process 800 may include causing data associated with the personalized glucose ranges to be presented within a user interface to the user. For example, the nutritional guidance service 112 generates user scores 116 (e.g., guidance data) and provides the user scores 116 to a user interface display service 118 to provide to the user via UI 134. The user scores 116 displayed in UI 134 may be used by an individual to help them understand how they can improve their diet. For instance, a user may receive the user scores 116 by viewing UI 134 on a mobile device, or some other device, to readily determine how good/bad a particular food and/or meal is for them.

FIG. 9 illustrates a flow diagram of an example process 900 for generating a biomarker score for a food item. The order in which the operations or steps are described is not intended to be construed as a limitation, and any number of the described operations may be combined in any order and/or in parallel to implement process 900. The operations described with respect to the process 900 are described as being performed by a nutritional service. However, it should be understood that some or all of these operations may be performed by some or all of components, devices, and/or systems described herein.

At block 902, the process 900 may include accessing glucose data associated with a user, the glucose data being obtained at least partly from a continuous glucose monitor (CGM).

At block 904, the process 900 may include generating personalized glucose ranges, based at least in part on the glucose data, wherein the personalized glucose ranges are personalized for the user.

At block 906, the process 900 may include receiving at least one input identifying at least one food item. For example, biomarker prediction service 104 may generate a prediction of the postprandial response of the user's biomarker to the food 142 being evaluated

At block 908, the process 900 may include determining food data associated with the at least one food item, the food data including at least one of a sugar content, a carbohydrate content, a glycemic index of the carbohydrate, a fiber content, an amount of processing, or a food category. For example, the nutritional guidance service 112 also utilizes non-biomarker inputs such as but not limited to food data 115 such as sugar content, carbohydrate content, glycemic index of the carbohydrate, fiber content, amount of processing on the food (e.g., high, medium, low), food category and the like. In some examples the nutritional guidance service uses information about the individual to determine personalizing weighting 114 for combining these scores, which determine how to combine the different biomarker scores of the biomarker scoring service 106 and food data, 113 into a single score.

At block 910, the process 900 may include determining health data associated with a user, based at least in part on the personalized glucose ranges.

At block 912, the process 900 may include generating a postprandial response prediction associated with the at least one food item based at least in part on the food data and the health data. For example, the nutritional guidance service 112 may receive one or more inputs (e.g., the glucose rise linked to foods). In this example, the nutritional guidance service 112 generates a prediction of the biomarker of interest using biomarker prediction service 104 and a score for that biomarker using the biomarker scoring service 106. However, in other examples where more than one aspect of the food is calculated, nutritional guidance service 112 predicts many different biomarkers (e.g., biomarkers 1-N) of the assessed aspects (glucose response, blood fat response, and the like), scores each biomarker change individually using the biomarker scoring service 106 and weights the scores so that the scores are comparable and then combined into a single score using food scoring service 108.

At block 914, the process 900 may include generating at least one biomarker score associated with the at least one food item based at least in part on the postprandial response prediction. For example, biomarker prediction service 104 generates a prediction of the postprandial response of the user's biomarker to the food 142 being evaluated. The biomarker scoring service 106 generates scores that are personalized for a particular user. The scores generated by the biomarker scoring service 106 are related to the predicted health impact of these biomarker changes on the particular user. For example, a small rise in blood fat after a meal might have no negative impact on health and so the score for blood fat might be at the best level for that meal but a large rise in blood fat 6 hours after a meal might be detrimental to health and lead to a bad score for blood fat for that meal for that person. The nutritional service 120 may then use these biomarker scores to generate food scores for one or more foods/combination of foods and/or meals to make individual food recommendations by generating personalized meal scores for any meal using food scoring service 108. In some examples, the food scoring service 108 uses personalized weighting/scores 114 to determine how to weight the different biomarker scores when combining them to generate a single meal score. For example, the food scoring service might need to combine a blood glucose score, a blood fat score and a gut health score, and might do so by weighting each of these scores and then combining them.

At block 9116, the process 900 may include causing the at least one biomarker score to be presented within a user interface to the user. For example, the personalized impact of the food 142 being evaluated and any associated recommendation may be in the form of the user scores 116 and provided, via the UI display service 118, to the UI 134 and/or the application 136 of the computing device 132 for presentation to the user.

Based on the foregoing, it should be appreciated that technologies for generating personalized glucose ranges have been presented herein. Moreover, although some of the subject matter presented herein has been described in language specific to computer structural features, methodological acts and computer readable media, it is to be understood that the invention defined in the appended claims is not necessarily limited to the specific features, acts, or media described herein. Rather, the specific features, acts and media are disclosed as example forms of implementing at least some of the claims.

The subject matter described above is provided by way of illustration only and should not be construed as limiting. Furthermore, the claimed subject matter is not limited to implementations that solve any or all disadvantages noted in any part of this disclosure. Various modifications and changes may be made to the subject matter described herein without following the examples and applications illustrated and described, and without departing from the true spirit and scope of the present invention, which is set forth in the following claims.

Although embodiments have been described in language specific to structural features and/or methodological acts, it is to be understood that the disclosure is not necessarily limited to the specific features or acts described. Rather, the specific features and acts are disclosed herein as illustrative forms of implementing the claimed subject matter. Each claim of this document constitutes a separate embodiment, and embodiments that combine different claims and/or different embodiments are within the scope of the disclosure and will be apparent to those of ordinary skill in the art after reviewing this disclosure.

### Example clauses

A: A method, comprising accessing glucose data associated with a user, generating personalized glucose ranges, based at least in part on the glucose data, wherein the personalized glucose ranges are personalized for the user, and causing data associated with the personalized glucose ranges to be presented within a user interface to the user.
B: The system of clause A, further comprising receiving the glucose data from a continuous glucose monitoring (CGM) device associated with the user.
C: The article of manufacture of clause A, wherein the glucose data is collected during a calibration period that lasts a number of days.
D: The article of manufacture of clause A, further comprising receiving nutritional data associated with the user, wherein the nutritional data indicates at least one food item that the user consumed during a calibration period.
E: The article of manufacture of clause A, further comprising receiving context data associated with the user, wherein the context data indicates at least one of a time of day of consumption, a previously eaten food item, an amount of exercise, and/or an amount of sleep associated with the user during a calibration period.
F: The article of manufacture of clause E, wherein the context data is received from the user via an application stored on a user device associated with the user.
G: The article of manufacture of clause A, further comprising determining a time of day in which a food item was eaten by the user, determining that a glucose level changes to at least one of above a first threshold value or below a second threshold value during the time of day, and associating the glucose level change with the food item in response to the glucose level changing when the food item was eaten.
H: The article of manufacture of clause A, further comprising receiving at least one input identifying at least one food item, determining food data associated with the at least one food item, determining health data associated with the user, generating a postprandial glucose response prediction associated with the at least one food item based at least in part on the food data and the health data, and causing the postprandial glucose response prediction to be presented within a user interface to the user in relation to their personalized glucose ranges.
I: The article of manufacture of clause A, wherein the food data includes at least one of sugar content, carbohydrate content, glycemic index of the carbohydrate, fiber content, an amount of processing, or a food category.
J: The article of manufacture of clause A, wherein health data includes at least one of a blood glucose control score, a blood fat control score, or a gut health score.
K: A system comprising one or more processors, and one or more non-transitory computer-readable media comprising instructions that, when executed by the one or more processors, cause the one or more processors to perform operations comprising accessing glucose data associated with a user, generating personalized glucose ranges, based at least in part on the glucose data, wherein the personalized glucose ranges are personalized for the user, and causing data associated with the personalized glucose ranges to be presented within a user interface to the user.
L: The system of clause L, wherein the personalized glucose ranges are further based at least in part on accessing at least one of personalized health score data, single meal data, single food data, or combination of meal data.
M: The system of clause L, further comprising receiving nutritional data associated with the user, wherein the nutritional data indicates at least one food item that the user consumed during a calibration period, identifying a glucose change associated with the user at a time in which the at least one food item was consumed, and generating a biomarker score associated with the at least one food item based at least in part on the glucose change.
N: The system of clause L, further comprising causing an indication of an upper limit and a lower limit of the glucose ranges to be presented via the user interface of the user.
O: The system of clause L, wherein the data associated with the personalized glucose ranges includes a line graph indicating a glucose level via a y-axis over a period of time via an x-axis.
P: A method comprising accessing glucose data associated with a user, the glucose data being obtained at least partly from a continuous glucose monitor (CGM), generating personalized glucose ranges, based at least in part on the glucose data, wherein the personalized glucose ranges are personalized for the user; receiving at least one input identifying at least one food item, determining food data associated with the at least one food item, the food data including at least one of a sugar content, a carbohydrate content, a glycemic index of the carbohydrate, a fiber content, an amount of processing, or a food category, determining health data associated with the user based at least in part on the personalized glucose ranges, generating a postprandial response prediction associated with the at least one food item based at least in part on the food data and the health data, generating at least one biomarker score associated with the at least one food item based at least in part on the postprandial response prediction, and causing the at least one biomarker score to be presented within a user interface to the user.
Q: The method of clause P, further comprising applying at least one weight to at least one of the health data, wherein the at least one biomarker score is generated based at least in part on the at least one weight.
R: The method of clause P, wherein the at least one biomarker score is generated by a machine learning model.
S: The method of clause P, further comprising generating the at least one biomarker score based on accessing at least one of a family health history associated with the user, measures of blood chemistry taken in a fasting state associated with the user, or measures of blood chemistry that do not change postprandially associated with the user.
T: The method of clause P, further comprising receiving nutritional data associated with the user, wherein the nutritional data indicates at least one food item that the user consumed during a calibration period when the glucose data is collected.

## Claims

1. A method, comprising:
accessing glucose data associated with a user;
generating personalized glucose ranges, based at least in part on the glucose data, wherein the personalized glucose ranges are personalized for the user; and
causing data associated with the personalized glucose ranges to be presented within a user interface to the user.

2. The method of claim 1, further comprising receiving the glucose data from a continuous glucose monitoring (CGM) device associated with the user.

3. The method of claim 1 or 2, wherein the glucose data is collected during a calibration period that lasts a number of days.

4. The method of any of the preceding claims, further comprising receiving nutritional data associated with the user, wherein the nutritional data indicates at least one food item that the user consumed during a calibration period, preferably a calibration period when the glucose data is collected.

5. The method of any of the preceding claims, further comprising receiving context data associated with the user, preferably received from the user via an application stored on a user device associated with the user, wherein the context data indicates at least one of a time of day of consumption, a previously eaten food item, an amount of exercise, and/or an amount of sleep associated with the user during a calibration period.

6. The method of any of the preceding claims, further comprising:
determining a time of day in which a food item was eaten by the user;
determining that a glucose level changes to at least one of above a first threshold value or below a second threshold value during the time of day; and
associating the glucose level change with the food item in response to the glucose level changing when the food item was eaten.

7. The method of any of the preceding claims, further comprising:
receiving at least one input identifying at least one food item;
determining food data associated with the at least one food item;
determining health data associated with the user preferably based at least in part on the personalized glucose ranges;
generating a postprandial glucose response prediction associated with the at least one food item based at least in part on the food data and the health data; and
causing the postprandial glucose response prediction, or at least one biomarker score associated with the at least one food item based at least in part on the postprandial response prediction, to be presented within a user interface to the user, preferably in relation to their personalized glucose ranges.

8. The method of any of the preceding claims, wherein the food data includes at least one of sugar content, carbohydrate content, glycemic index of the carbohydrate, fiber content, an amount of processing, or a food category.

9. The method of any of the preceding claims, wherein health data includes at least one of a blood glucose control score, a blood fat control score, or a gut health score.

10. The method of any of the preceding claims, wherein the personalized glucose ranges are further based at least in part on accessing at least one of personalized health score data, single meal data, single food data, or combination of meal data.

11. The method of any of the preceding claims, further comprising causing an indication of an upper limit and a lower limit of the glucose ranges to be presented via the user interface of the user, and/or wherein the data associated with the personalized glucose ranges includes a line graph indicating a glucose level via a y-axis over a period of time via an x-axis.

12. The method of any of the preceding claims, further comprising applying at least one weight to at least one of the health data, wherein the at least one biomarker score is generated based at least in part on the at least one weight.

13. The method of any of the preceding claims, wherein the at least one biomarker score is generated by a machine learning model.

14. The method of any of the preceding claims, further comprising generating the at least one biomarker score based on accessing at least one of a family health history associated with the user, measures of blood chemistry taken in a fasting state associated with the user, or measures of blood chemistry that do not change postprandially associated with the user.

15. A system comprising:
one or more processors; and
a computer program comprising instructions that, when executed by the one or more processors, cause the one or more processors to perform the method of any of the preceding claims.
